# EUROPEAN PATENT APPLICATION

(11) **EP 1 911 424 A1**
(43) Date of publication of application: **16.04.2008**
(21) Application number: 07020035.7
(22) Date of filing: 12.10.2007
(51) Int. Cl.: A61F 7/08

(54) **Heating body**

(30) Priority: 13.10.2006 JP 2006279742
(71) Applicant: JAPAN PIONICS CO., LTD., Minato-ku, Tokyo (JP)
(72) Inventor: Asao, Ajiri, Hiratsuka-shi Kanagawa (JP); Yoshiki, Matsumoto, Hiratsuka-shi Kanagawa (JP)
(74) Representative: Leifert & Steffan

(57) **Abstract**

A heating body is provided. The heating body utilizes a heating composition that generates heat when it is brought into contact with oxygen in the air and being controlled easily and correctly in heating temperatures (heating properties). (1) The heating body includes a heating composition housed in a flat air permeable flat bag using an air permeable packaging material formed on one side or both sides thereof. In the heating body, air permeable pores are formed on the packaging material by irradiation of laser rays. The air permeable pores have diameters or circle-corresponding diameters of 0.005 to 2.0 mm. A distance between the centers of the air permeable pores adjacent to each other is 0.1 to 20 mm. The percentage of the area of the air permeable pores to the total area of the laminate sheet is 0.001 to 5%. (2) In the heating body according to the above Item 1, the heating composition may be held by a sheet-like support. (3) A plurality of heating cells made of a heating composition to be heated in contact with oxygen in the air are housed in a plurality of air permeable packaging material, respectively. The spaces are formed by two sheets, where the air permeable packaging material is formed on one side or both sides thereof. In addition, air permeable pores are formed on the air permeable packaging material by irradiation of laser rays on a lamination sheet having a plastic film. The air permeable pores have diameters or circle-corresponding diameters of 0.005 to 2.0 mm. The distance between the centers of the air permeable pores adjacent to each other is 0.1 to 20 mm. The percentage of the area of the air permeable pores to the total area of the laminate sheet is 0.001 to 5%.

## Description

### BACKGROUND OF THE INVENTION

### 1. Field of the Invention

The present invention relates to a heating body, and more specifically, to a heating body excellent in uniformities of the sizes and shapes of air permeable pores and capable of easily and correctly controlling exothermic characteristics thereof.

### 2. Description of the Related Art

Heretofore, heating compositions housed in air permeable flat bags have been used as disposable body warmers (heating bodies). The heating composition contains a mixture of an oxidizable metal, activated carbon, inorganic electrolyte, water, water-retaining agent, and the like and is provided for generating heat by being in contact with oxygen in air. In addition, the flat bag is made of a packaging material with a plurality of pores, pinholes, or the like on one side or both sides thereof. Suchbodywarmers include, for example, those disclosed in Japanese Utility Model Application Laid-open No. Sho 59-178548 and Japanese Utility Model Application Laid-open No. Sho 60-58125. In the body warmer, the air permeable flat bag is hermetically sealed in a non-air permeable outer bag until it is used in order to prevent the heating composition from being brought into contact with outside air and to prevent the water from evaporating to diffuse outside.

Further, as the disposable body warmers as described above, sheet-shaped disposable body warmers are also used. In this case, the heating composition is retained in spaces of a sheet-like support such as a non-woven cloth, and then housed in the flat bag, thereby preventing the heating composition from biasing during the use. Such disposable body warmers include one disclosed in Japanese Patent Application Laid-open No. Hei 03-152894.

Various kinds of those body warmers are commercially available in the market. That is, the heating properties of the body warmers are set depending on portions to be applied and designed to be used on the body, in a pocket, in a footwear, and the like. Some body warmers are provided with pressure-sensitive adhesive layers so that the user can easily put the body warmer on the body at the time of use.

Further, as disposable body warmers other than the above-mentioned body warmer, there are provided body warmers developed with an object of being fitted to a bendable portion such as an elbow or a knee to be used. There are listed, for example, as disclosed in Japanese Utility Model Application Laid-open No. Hei 06-26829, a disposable body warmer in which a heating portion filled with a heating agent (heating composition) is divided into a plurality of sections by a seal portion, and as disclosed in Japanese Utility Model Application Laid-open No. Hei 06-61222, an expandable disposable body warmer having a structure in which a disposable body warmer is provided to a desired surface of an expandable material, thereby fitting a body.

Among the air permeable packaging materials used in disposable body warmers as described above, those with relatively small air permeable pores include a packaging material in which a plurality of pores are formed by randomly stacking and hot-pressing synthetic resin fibers; and a packaging material in which a plurality of pores are formed by dispersing an inorganic material such as calcium carbonate, silica dioxide, or aluminum dioxide, in a synthetic resin, followed by extrusion-molding into a sheet. In addition, the packaging materials provided with relatively large air permeable pores include those in which pinholes are formed in plastic films.

However, the formation of flat bags or pinholes, which have been conventionally performed in the art, dos not sufficiently retain the sizes and shapes of pores in uniform. When the amount of air captured in the permeable flat bag is less than the predetermined range, a disadvantage occurs in that the disposable body warmer can be insufficiently warmed. In contrast, when the amount of air captured in the permeable flat bag is more than the predetermined range, a disadvantage occurs in that the disposable body warmer can be excessively warmed and the user may get burned. Thus, giving priority to safety, a disposal body warmer (heating body) typically set to a relatively lower heating temperature has been produced.

Therefore, the present invention intends to provide a disposable body warmer (heating body), which may be produced as any kind of the aforementioned disposable body warmers (heating bodies), with abilities of easily and correctly controlling heating temperatures (heating properties) thereof.

As a result of intensive studies for solving the above-mentioned problems, the inventors of the present invention have found that a heating body, which can be extensively improved in uniformities of the sizes and shapes of air permeable pores, and easily and correctly control heating temperature (heating properties), can be obtained by the use of an air permeable packaging material made of a lamination sheet having a plastic film in which air permeable pores are formed by irradiation of laser rays as an air permeable packaging material used in the disposable body warmer (heating body).

### SUMMARY OF THE INVENTION

In other words, the present invention relates to a heating body, including: a heating composition to be heated in contact with oxygen in the air; an air permeable packaging material in which air permeable pores are formed in a laminate sheet including a plastic film by irradiation of laser rays; and a flat air permeable bag having the air permeable packing material on one side or both sides thereof, in which: the heating composition is housed in the flat air permeable bag; the air permeable pores have diameters or circle-corresponding diameters of 0.005 to 2.0 mm; a distance between the centers of the air permeable pores adjacent to each other is 0.1 to 20 mm; and a percentage of the area of the air permeable pores to the total area of the laminate sheet is 0.001 to 5%.

Further, the present invention relates to a heating body including: a heating composition to be heated in contact with oxygen in the air; a sheet-like support which retains the heating composition; an air permeable packaging material in which air permeable pores are formed in a laminate sheet including a plastic film by irradiation of laser rays; and a flat air permeable bag used for one side or both sides which houses the air permeable packaging material, in which the air permeable pores have diameters or circle-corresponding diameters of 0.005 to 2.0 mm; the distance between the centers of the air permeable pores adjacent to each other is 0.1 to 20 mm; and the percentage of the area of the air permeable pores to the total area of the laminate sheet is 0.001 to 5%.

Still further, the present invention relates to a heating body, including: a plurality of heating cells made of a heating composition to be heated in contact with oxygen in the air; an air permeable packaging material in which air permeable pores are formed in a laminate sheet including a plastic film by irradiation of laser rays; and a plurality of spaces constructed of two sheets having the air permeable packing material on one side or both sides, in which: the plurality of the heating cells are housed in the plurality of spaces one by one; the air permeable pores have one of diameters and circle-corresponding diameters of 0.005 to 2.0 mm; a distance between the centers of the air permeable pores adjacent to each other is 0.1 to 20 mm; and a percentage of the area of the air permeable pores to the total area of the laminate sheet is 0.001 to 5%.

The heating body of the present invention includes an air permeable packaging material in which air permeable pores are formed by irradiation of laser rays and is excellent in uniformities of the sizes and shapes of air permeable pores, whereby the heating temperature (heating properties) of the heating body can be easily and correctly controlled.

In addition, in the heating body of the present invention, patterns of air permeable pores can be easily changed by a program because the air permeable pores are formed by laser rays. For example, the heating body of the present invention can be produced while changing the sizes, shapes, and arrangement patterns of air permeable pores freely formed in the air permeable packaging material by providing the heating body with a heat gradient so that, for example, the central part thereof is heated to high temperature and the peripheral part thereof is cooled to low temperature.

### BRIEF DESCRIPTION OF DRAWINGS

In the accompanying drawings:
FIG. 1 is a sectional view illustrating an example of a heating body according to the present invention (first structure);
FIG. 2 is a perspective view illustrating an example of the heating body of FIG. 1 being sealed in a non-air permeable flat bag (partially cutaway perspective diagram);
FIG. 3 is a sectional view illustrating an example of a heating body according to the present invention (second structure);
FIG. 4 is a perspective view illustrating an example of the heating body of FIG. 3 being sealed in a non-air permeable flat bag (partially cutaway perspective diagram);
FIG. 5 is a sectional view illustrating an example of a heating body according to the present invention (third structure);
FIG. 6 is a perspective view illustrating an example of the heating body of FIG. 5 being sealed in a non-air permeable flat bag (partially cutaway perspective diagram); and
FIG. 7 is a perspective view illustrating an example of a packaging material used in the heating body of the present invention.

### DETAILED DESCRIPTION OF THE PREFERRED EMBODIMENTS

The configuration of the heating body of the present invention is applied to a heating body that utilizes a heating composition to be heated in contact with oxygen in the air. Further, the heating body of the present invention is not limited to the disposable body warmer and may be applied as a therapeutic heating tool for medical purposes.

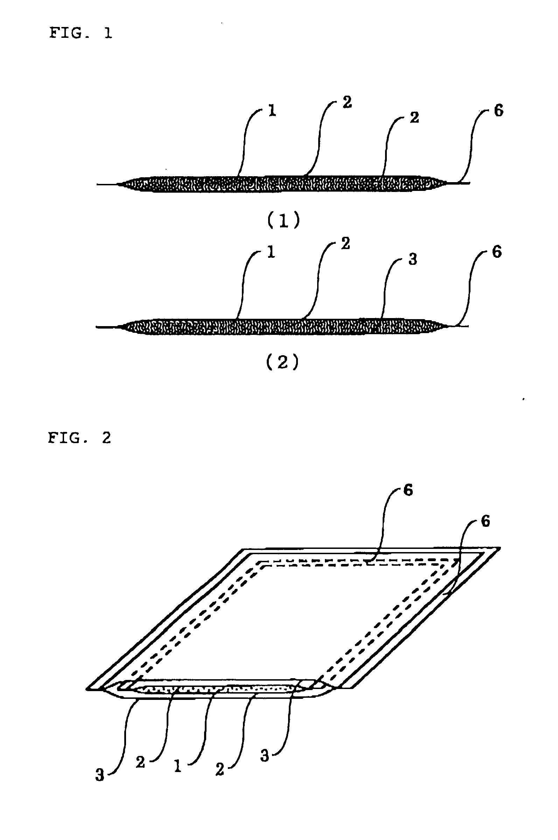

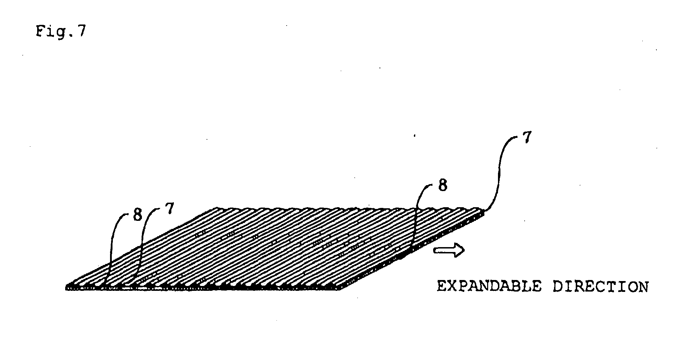

Hereinafter, the heating body of the present invention will be described in detail with reference to FIGS. 1 to 7, but not limited thereto. FIGS. 1, 3, and 5 are sectional views illustrating the respective examples of the heating body of the present invention. FIGS. 2, 4, and 6 are perspective views (partially cutaway perspective views) illustrating the examples of the heating bodies of FIGS. 1, 3, and 5 being hermetically sealed in a flat bags with no air permeability, respectively. FIG. 7 is a perspective view illustrating an example of a packaging material used in the heating body of the present invention.

In a heatingbody according to the first structure of the present invention, as shown in FIG. 1, a heating composition 1 to be heated in contact with oxygen in the air is housed in an air permeable flat bag having an air permeable packaging material 2 on one or both sides thereof. The air permeable packaging material 2 is made of a lamination sheet having a plastic film. In addition, air permeable pores are formed in the air permeable packaging material 2 by irradiation of laser rays.

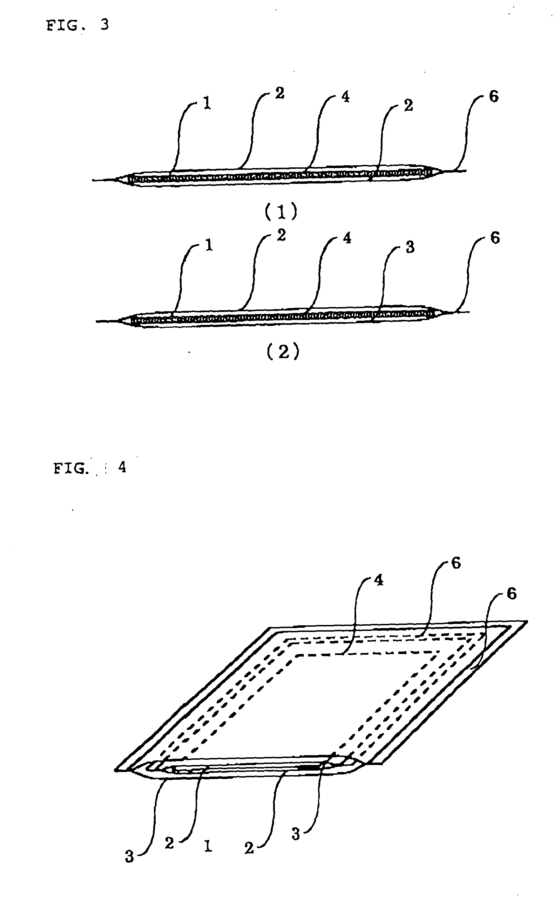

In a heating body according to the second structure of the present invention, as shown in FIG. 3, a heating composition 1 to be heated in contact with oxygen in the air is held on a sheet-like support 4. The support 4 is housed in an air permeable flat bag having an air permeable packaging material 2 on one or both sides thereof. The air permeable packaging material 2 is made of a lamination sheet having a plastic film. In addition, air permeable pores are formed in the air permeable packaging material 2 by irradiation of laser rays.

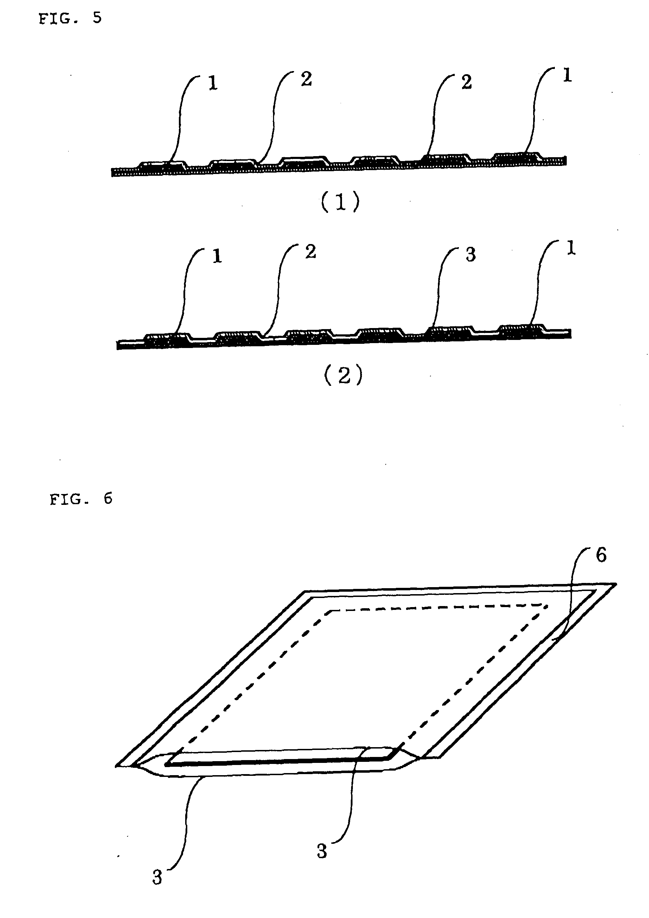

In a heating body according to the third structure of the present invention, as shown in FIG. 5, a plurality of heating cells each made of a heating composition 1 to be heated in contact with oxygen in the air are housed in a plurality of spaces one by one. The spaces are formed by two sheets where an air permeable packaging material 2 made of a lamination sheet having a plastic film is used on one or both sides thereof. As the air permeable packaging material 2, packaging material in which air permeable pores are formed by irradiation of laser rays is used.

It should be noted that, in any of those structures, when the both sides of the packaging material are air permeable packaging material, one having air permeable pores formed in only one side by irradiation of laser rays may be used.

A material of the air permeable packaging material used in the heating body according to each of the first and second structures of the present invention is any of lamination sheets includingplastic films. Those lamination sheets include, for example, (1) a lamination sheet of thermal adhesive component layer/plastic film/thermal adhesive component layer/non-woven cloth or (2) a lamination sheet of (mixture film of thermal adhesive component with plastic component)/non-woven cloth. It should be noted that, examples of the thermal adhesive component include polyethylene and polypropylene, and that the thermal adhesive component layer is a layer containing one of the thermal adhesive components or a combination of two or more thermal adhesive components. In a case of the above (1), the thermal adhesive components usedmaybe identical or different from each other. The plastic components include a polyethylene, a polypropylene, a polyester, andanylon. Theplastic film may be, for example, a film made of at least one selected from a polyethylene film, a polypropylene film, a polyester film, and a nylon film, each contains one of the above plastic components.

Further, the material of the air permeable packaging material used in the heating body according to the third structure of the present invention is any of lamination sheets including plastic films. Examples of the lamination sheet include (1) a lamination sheet of thermal adhesive plastic component layer/ (one or more kinds of different films selected from a plastic film, a rubber film, and a mixture film of a plastic component and a rubber component)/thermal adhesive plastic component layer/non-woven cloth, (2) a lamination sheet of (a mixture film of a thermal adhesive plastic component and a plastic component)/non-woven cloth, (3) lamination sheet of (a mixture film of a thermal adhesive plastic component and a rubber component)/non-woven cloth, and (4) a lamination sheet of (a mixture film of thermal adhesive plastic component, a plastic component, and a rubber component)/non-woven cloth. Examples of the thermal adhesive plastic components include thermal adhesive plastics such as a polyethylene resin, a polypropylene resin, vinyl chloride resin, an ABS resin, a polyethylene terephthalate resin, and a polyamide resin and thermal adhesive fluorine resins. Of those, a low-density polyethylene resin and a polypropylene resin are preferable. The thermal adhesive plastic component layer is one containing any of those thermal adhesive plastic components alone or in combination, and includes, for example, a layer constructed of a low-density polyethylene resin and a polyethylene resin; a layer constructed of a low-density polyethylene rein and a polypropylene resin. The rubber component may be, for example, a polybutadiene rubber, a polyisoprene rubber, a styrene-butadiene rubber, or a polyurethane rubber, each of which contains a monomer component such as butadiene, isoprene, styrene, or urethane.

In the present invention, even in the case of any structure as described above, an air permeable packaging material is typically provided such that air permeable pores are formed in any of those packaging materials by irradiation of laser rays. However, for example, an air permeable packaging material may be formed such that air permeable pores are formed in a plastic film by irradiation of laser rays and the plastic film is then laminated on an air permeable non-woven cloth.

In the present invention, a non-air permeable packaging material 3 may be, for example, any material of the above packaging material directly used without the formation of air permeable pores. In addition, the thermal adhesive side and its opposite side of the non-air permeable packaging material 3 of the present invention may be provided with an adhesive layer for the use of the heating body to be adhered on the body. The adhesive layer may be covered with release paper or the like until it is used.

In the heating body of the present invention, amethodof forming air permeable pores by irradiation of laser rays on a lamination sheet including a plastic film as described above is not specifically limited. For example, a UV laser or a carbon dioxide gas laser can be used. The irradiation of laser rays is typically carried out at a wavelength of 0.1 to 1, 000 µm and an output power of 1 to 1, 000 W. The air permeable pore is typically in the form of a circle or may be in the form of an ellipsoid, a triangle, a square, a rectangle, a rhomboid, a trapezoid, or a polygon.

The size of the air permeable pore is, in the case of a circle, 0.005 to 2.0 mm, preferably 0.05 to 0.75 mm. In the case of another shape except the circle, the diameter thereof corresponding to a circle is the same as the above diameter of the circle. In this case, the term "diameter thereof corresponding to a circle" denotes the diameter of a circle having the same area as that of one air permeable pore. In addition, the distance between the centers of two air permeable pores adjacent to each other is 0.1 to 20 mm, preferably 1 to 7.5 mm, but cannot be completely defined because of dependence on the sizes of the respective air permeable pores.

The air permeating pores maybe arranged without any particular limitation. They may be suitably arranged depending on the usage. For example, the air permeating pores may be formed in the whole, center, peripheral, or part of the lamination sheet, and typically and preferably, uniformly formed on the whole surface of the lamination sheet. The number of air permeable pores is typically 100 to 1,000,000 used for the formation of one laminate sheet, though depending on the size of air permeating pores and the size of the laminate sheet. In addition, a ratio of the area of the air permeating pore to the total area of the lamination sheet is 0.001 to 5%, preferably 0.025 to 0.75%.

A heating composition to be heated in contact with oxygen in the air, which can be used in each of the heating bodies according to the first and second structures of the present invention, may be a combination of an oxidizable metal, an activated carbon, an inorganic electrolyte, water, a water-retaining polymer agent, and the like.

The oxidizable metal powder used may be iron powder, aluminum power, or the like. In general, the iron power is used. Besides, reduced iron powder, atomized iron powder, electrolytic iron powder, or the like may be used. The activated carbon may be used as a water-retaining agent as well as a reaction auxiliary agent and may be typically coconut shell charcoal, wood powder charcoal, peat charcoal, or the like. The inorganic electrolytes preferably include chlorides of alkali metals, alkaline earth metals, and heavy metals and sulfates of alkali metals, such as sodium chloride, potassium chloride, calcium chloride, magnesium chloride, ferric chloride, and sodium sulfate. The percentages of those typical components are 20 to 80 wt% of oxidizable metal, 1 to 20 wt% of activated carbon, 1 to 20 wt% of inorganic electrolyte, 5 to 50 wt% of water, and 1 to 20 wt% of a water-retaining agent.

Further, in the heating body according to the third structure of the present invention, the heating composition used may be the same as one described above. However, as shown in FIG. 5, a plurality of heating cells provided by independently arranging the respective heating compositions needs to be fixed on the surface of the packaging material. Thus, a preferable heating composition may contain a thickener in addition to the above components so as to be preferably fixed on the surface of the packaging material by coating or printing. The percentages of those typical components are 20 to 80 wt% of oxidizable metal, 1 to 10 wt% of activated carbon, 1 to 10 wt% of inorganic electrolyte, 5 to 50 wt% of water, 1 to 10 wt% of water-retaining agent, and 0.1 to 10 wt% of thickener.

The heating body of the first structure of the present invention is obtained as one as shown in FIG. 1. In other words, typically, laser rays are irradiated on the above lamination sheet having a plastic film to form air permeable pores, thereby providing an air permeable packaging material. Then, two air permeable packaging materials or the air permeable packaging material and a packaging material with no air permeability are laminated together so that the thermal adhesive layers are inwardly faced, while the periphery thereof is thermally fused to forma bag-like product. Subsequently, the above heating composition is filled in the bag, thereby obtaining a heating body as shown in FIG. 1. The heating body is further sealed off in a non-air permeable flat bag as shown in FIG. 2 to prevent the heating body 1 from spreading to the outside through the evaporation of water while shutting out the outside before it is used.

In the heating body according to the second structure of the present invention, the sheet-like support that retains the heating composition in the spaces can be obtained by, for example, dispersing on the non-woven cloth thermal adhesive powder, such as an ethylene/vinyl acetate copolymer resin in addition to the above-mentioned heating composition, laminating a non-woven cloth similar to one described above thereon, and heating and compression-bonding the resultant by a mold-compressor. The heating body according to the second structure of the present invention is obtained as one as shown in FIG. 3. In other words, typically, laser rays are irradiated on the above lamination sheet having a plastic film to form air permeable pores, thereby providing an air permeable packaging material. Then, two air permeable packaging materials or the air permeable packaging material and a packaging material with no air permeability are laminated together so that the thermal adhesive layers are inwardly faced, while the periphery thereof is thermally fused to form a bag-like product. Subsequently, the above sheet-like support is housed in the bag, thereby obtaining a heating body as shown in FIG. 3. The heating body is further sealed off in a non-air permeable flat bag as shown in FIG. 4 to prevent the heating body 1 from spreading to the outside through the evaporation of water while shutting out the outside before it is used.

The heating body according to the third structure of the present invention is obtained as one as shown in FIG. 5. In other words, typically, laser rays are irradiated on the above lamination sheet having a plastic film to form air permeable pores, thereby providing an air permeable packaging material. Then, the above thermal composition is arranged by applying or printing on the surface of the air permeable packaging material or a packaging material with no air permeability so that the thermal composition becomes a plurality of flat heating cells. Further, on the resultant, an additional air permeable packaging material or an additional packaging material with no air permeability is laminated so that the thermal adhesive side thereof is located inside and then heated and compression-bonded, thereby obtaining the heating body as shown in FIG. 5. However, at least one of two packaging materials is an air permeable packaging material. The heating body is further sealed off in a non-air permeable flat bag as shown in FIG. 6 to prevent the heating body 1 from spreading to the outside through the evaporation of water while shutting out the outside before it is used.

### Examples

Next, examples of the present invention will be described in detail, but the present invention is not limited by the examples.

### Example 1

### (Manufacture of air permeable packaging material)

A packaging material was continuously pulled out of a rolled laminate packaging material made of a commercially-available sheet of (a mixture film of thermal adhesive plastic component and polyethylene)/non-woven cloth. Then, the packaging material was irradiated with UV to form air permeable pores, thereby manufacturing an air permeable packaging material. Uniform air permeable pores with a diameter of 0.5 mm were formed in the whole surface of the air permeable packaging material at intervals of about 6 mm long and about 6 mm wide. Further, the air permeable packaging material is cut into pieces of 100 mm x 150 mm. Subsequently, the air permeable packaging material was subjected to the measurement of Gurley air permeability of the air permeable packaging product, resulting in 23 seconds/100 ml.

### (Manufacture of heating body)

Under a nitrogen gas atmosphere, a heating composition was prepared by mixing 50 wt% of iron powder, 10 wt% of activated carbon, 3 wt% of salt (inorganic electrolyte), 25 wt% of water, and 12 wt% of a water-retaining agent. The above two air permeable packaging materials were laminated so that thermal adhesive surfaces thereof were facing inside each other. The periphery of the resulting laminate was heated and compression-fused to make the laminate into a bag shape, while 40 g of a heating composition was filled to manufacture a thermal body as shown in FIG. 1. After that, the heating body was enclosed in a non-air permeable flat bag (130 mm x 180 mm in size).

### (Examination of variations of heating properties of heating body)

One hundred heating bodies described above were manufactured and were left for one day and one night in a room at 25°C. Next, for those one hundred heating bodies, heating body was taken out from each non-air permeable flat bag and then left standing on a cushion in a room at 25°C. At this time, the thermal properties of the heating body were determined and the variations thereof were then examined. As a result, with respect to the maximum temperature in the 100 bags, it was confirmed that the maximum value was 68°C, the minimum value was 63°C, and the difference therebetween was 5°C.

### Example 2

### (Manufacture of heating body)

In the manufacture of the air permeable packaging material of Example 1 of the present invention, an air permeable packaging material was manufactured in the same way as that of Example 1, except that the diameter of the air permeable pore was 0.1 mm and the intervals were 3 mm long and 6 mm wide. As a result of determining the Gurley air permeability of the air permeable packaging material, 32 seconds/100 ml was obtained. The air packaging material (100 mm x 150 mm) and the packaging material with no air permeability (100 mm x 150 mm) before the formation of an air permeating pore were laminated together so that the thermal adhesive surfaces thereof were facing inside. The periphery of the laminate was heat-fused, thereby forming a bag. In addition, 40 g of a heating composition was filled in a manner similar to Example 1 of the present invention, thereby obtaining a heating body. After that, the heating body was sealed in a non-air permeable flat bag of 130 mm x 180 mm in size.

### (Examination of variations of heating properties of heating body)

One hundred heating bodies described above were manufactured and were left for one day and one night in a room at 25°C. Next, for those hundred heating bodies, heating body was taken out from each non-air permeable flat bag and then left standing on a cushion in a room at 25°C. At this time, the thermal properties of the heating body were determined and the variations thereof were then examined. As a result, with respect to the maximum temperature in the 100 bags, it was confirmed that the maximum value was 62°C, the minimum value was 58°C, and the difference therebetween was 4°C.

### Comparative Example 1

### (Manufacture of heating body)

A heating body was manufactured in the same way as that of Example 1 of the present invention, except that a commercially-available laminate packaging material made of (a mixture film of thermal adhesive plastic component and polyethylene)/non-woven cloth. As in the case of Example 1 of the present invention, circular air permeating pores made of pinholes of 0.5 mm in diameter were formed on the whole surface of the air permeable packaging material at intervals of about 6 mm long and 5 mm wide. However, the observation of the surface of the heating body with a magnifying glass confirmed that the sizes and shapes of air permeable pores were uneven, compared with the air permeable packaging material of Example 1 of the present invention. Subsequently, the heating body was sealed in a non-air permeable flat bag of 130 mm x 180 mm in size.

### (Examination of variations of heating properties of heating body)

One hundred heating bodies described above were manufactured and were left for one day and one night in a room at 25°C. Next, for those hundred heating bodies, heating body was taken out from each non-air permeable flat bag and then left standing on a cushion in a room at 25°C. At this time, the thermal properties of the heating body were determined and the variations thereof were then examined. As a result, with respect to the maximum temperature in the 100 bags, it was confirmed that the maximum value was 71°C, the minimum value was 59°C, and the difference therebetween was 12°C.

### Comparative Example 2

### (Manufacture of heating body)

A heating body was manufactured in the same way as that of Example 2 of the present invention, except that a commercially-available laminate packaging material made of (a mixture film of thermal adhesive plastic component and polyethylene)/non-woven cloth. As in the case of Example 2 of the present invention, circular air permeating pores made of pinholes of 0.1 mm in diameter were formed on the whole surface of the air permeable packaging material at intervals of about 3 mm long and 6 mm wide. However, the observation of the surface of the heating body with a magnifying glass confirmed that the sizes and shapes of air permeable pores were uneven, compared with the air permeable packaging material of Example 2 of the present invention. Subsequently, the heating body was sealed in a non-air permeable flat bag of 130 mm x 180 mm in size.

### (Examination of variations of heating properties of heating body)

One hundred heating bodies described above were manufactured and were left for one day and one night in a room at 25°C. Next, for those hundred heating bodies, heating body was taken out from each non-air permeable flat bag and then left standing on a cushion in a room at 25°C. At this time, the thermal properties of the heating body were determined and the variations thereof were then examined. As a result, with respect to the maximum temperature in the 100 bags, it was confirmed that the maximum value was 63°C, the minimum value was 50°C, and the difference therebetween was 13°C.

### Example 3

### (Manufacture of heating body)

A commercially-available non-woven fabric was cut into 80 mm x 130 mm sections. Under a nitrogen gas atmosphere, a mixture of 22 g of the same heating composition as that of Example 1 of the preset invention (but water was removed) and ethylene/vinyl acetate copolymer resin powder (thermal adhesive powder) was dispersed on the non-woven fabric. After that, an additional non-woven fabric of the same type as described above was laminated thereon and then heated and pressure-compressed by a mold compressor or the like, followed by dispersion of 8 g of water thereon. Consequently, a sheet-like support that retains a heating composition in a space was obtained. An air permeable packaging material (100 mm x 150 mm) manufactured in a manner similar to Example 1 of the present invention was laminated with a packaging material (100 mm x 150 mm) with no air permeability before the formation of air permeable pores so that thermal adhesive surfaces thereof were facing inside each other. Then, the periphery of the laminate was fused by heat, thereby forming into a bag, while the sheet-like support was housed in the bag. Consequently, a heating body was manufactured as shown in FIG. 3. After that, the heating body was sealed in a non-air permeable flat bag of 130 mm x 180 mm in size.

### (Examination of variations of heating properties of heating body)

One hundred heating bodies described above were manufactured and were left for one day and one night in a room at 25°C. Next, for those hundred heating bodies, heating body was taken out from each non-air permeable flat bag and then left standing on a cushion in a room at 25°C. At this time, the thermal properties of the heating body were determined and the variations thereof were then examined. As a result, with respect to the maximum temperature in the 100 bags, it was confirmed that the maximum value was 65°C, the minimum value was 60°C, and the difference therebetween was 5°C.

### Comparative Example 3

### (Manufacture of heating body)

A heating body was manufactured in the same way as that of Example 3 of the present invention, except that a commercially-available laminate packaging material made of (a mixture film of thermal adhesive plastic component and polyethylene)/non-woven cloth. As in the case of Example 3 of the present invention, circular air permeating pores made of pinholes of 0.5 mm in diameter were formed on the whole surface of the air permeable packaging material at intervals of about 6 mm long and 6 mm wide. However, the observation of the surface of the heating body with a magnifying glass confirmed that the sizes and shapes of air permeable pores were uneven, compared with the air permeable packaging material of Example 3 of the present invention. Subsequently, the heating body was sealed in a non-air permeable flat bag of 130 mm x 180 mm in size.

### (Examination of variations of heating properties of heating body)

One hundred heating bodies described above were manufactured and were left for one day and one night in a room at 25°C. Next, for those hundred heating bodies, heating body was taken out from each non-air permeable flat bag and then left standing on a cushion in a room at 25°C. At this time, the thermal properties of the heating body were determined and the variations thereof were then examined. As a result, with respect to the maximum temperature in the 100 bags, it was confirmed that the maximum value was 65°C, the minimum value was 54°C, and the difference therebetween was 11°C.

### Example 4

### (Manufacture of air permeable packaging material)

A mixture film of commercially-available thermal adhesive plastic component and rubber component was laminated with commercially-available non-woven fabric (polyester spun lace) and then sandwiched between two molds having the repetitive waveform, followed by thermal adhesive. Consequently, a stretchable laminate packaging material having a thermal adhesive surface with a repetitive waveform was obtained as shown in FIG. 7 (having stretching properties in the direction of the arrow in FIG. 7). Air permeable pores were formed in the laminate packaging material by irradiation of carbon dioxide gas laser, thereby manufacturing an air permeable packaging material. Uniform circular air permeable pores of 0.07 mm in diameter were formed on the whole surface of the air permeable packaging material at intervals of about 3 mm long and about 3 mm wide. Further, the air permeable packaging material was cut into a 100 mm x 150 mm sections. Further, the Gurley permeability of the air permeable packaging material was determined and resulted in 23 seconds/100 ml.

### (Manufacture of heating body)

Under a nitrogen gas atmosphere, 30 g of a coating liquid obtained by mixing 63.1 wt% of iron powder, 3.2 wt% of activated carbon, 1.9 wt% of salt (inorganic electrolyte), 26.5 wt% of water, 0.6 wt% of a water-retaining agent, and 4.7 wt% of a thickener was divided to be applied into six positions on a surface at a thermal adhesive-bondable surface side of the air permeable packaging material, thereby obtaining a heating cell. Subsequently, the same air permeable packaging material as one described above was laminated on the heating cell so that the thermal adhesive-bondable surfaces were facing inside each other, and then heat-compressed. Consequently, a heating body as shown in FIG. 5 was manufactured. After that, the heating body was sealed in a non-air permeable flat bag of 130 mm x 180 mm in size.

### (Examination of variations of heating properties of heating body)

One hundred heating bodies described above were manufactured and were left for one day and one night in a room at 25°C. Next, for those hundred heating bodies, heating body was taken out from each non-air permeable flat bag and then left standing on a cushion in a room at 25 °C. At this time, the thermal properties of the heating body were determined and the variations thereof were then examined. As a result, with respect to the maximum temperature in the 100 bags, it was confirmed that the maximum value was 53°C, the minimum value was 48°C, and the difference therebetween was 5°C.

### Comparative Example 4

### (Manufacture of heating body)

A heating body was manufactured in the same way as that of Example 4 of the present invention, except that the air permeable pores are formed by pinholes instead of air permeable pores formed by irradiation of carbon dioxide gas laser in the manufacture of the heating body in Example 4. As is the case of Example 4 of the present invention, circular permeable pores of 0.07 mm in diameter was formed in the whole surface of the air permeable packaging material at intervals of about 3 mm long and about 3 mm wide. However, the observation of the surface of the heating body with a magnifying glass confirmed that the sizes and shapes of air permeable pores were uneven, compared with the air permeable packaging material of Example 1 of the present invention. Subsequently, the heating body was sealed in a non-air permeable flat bag of 130 mm x 180 mm in size.

### (Examination of variations of heating properties of heating body)

One hundred heating bodies described above were manufactured and were left for one day and one night in a room at 25°C. Next, for those hundred heating bodies, heating body was taken out from each non-air permeable flat bag and then left standing on a cushion in a room at 25°C. At this time, the thermal properties of the heating body were determined and the variations thereof were then examined. As a result, with respect to the maximum temperature in the 100 bags, it was confirmed that the maximum value was 55°C, the minimum value was 42°C, and the difference therebetween was 13°C.

As described above, it becomes evident that the heating bodies of the examples of the present invention have extremely small variation of heating properties, compared with those of the comparative examples, and that the heating properties can be easily and correctly controlled.

## Claims

1. A heating body, comprising:
a heating composition to be heated in contact with oxygen in the air;
an air permeable packaging material in which air permeable pores are formed in a laminate sheet including a plastic film by irradiation of laser rays; and
a flat air permeable bag having the air permeable packaging material on one side or both sides, wherein:
the heating composition is housed in the flat air permeable bag;
the air permeable pores have one of diameters and circle-corresponding diameters of 0.005 to 2.0 mm;
a distance between the centers of the air permeable pores adjacent to each other is 0.1 to 20 mm; and
a percentage of the area of the air permeable pores to the total area of the laminate sheet is 0.001 to 5%.

2. A heating body according to claim 1, wherein the heating composition is held by a sheet-like support.

3. A heating body, comprising:
a plurality of heating cells made of a heating composition to be heated in contact with oxygen in the air;
an air permeable packaging material in which air permeable pores are formed in a laminate sheet including a plastic film by irradiation of laser rays; and
a plurality of spaces constructed of two sheets having the air permeable packagingmaterial on one side and both sides, wherein:
the plurality of heating cells are housed in the plurality of spaces one by one;
the air permeable pores have one of diameters and circle-corresponding diameters of 0.005 to 2.0 mm;
a distance between the centers of the air permeable pores adjacent to each other is 0.1 to 20 mm; and
a percentage of the area of the air permeable pores to the total area of the laminate sheet is 0.001 to 5%.

4. A heating body according to any one of claims 1 to 3, which is sealed in a non-air permeable flat bag.

5. A heating body according to claim 1 or 2, wherein the laminate sheet including the plastic sheet is made of one of (1) thermal adhesive component layer/plastic film/thermal adhesive component layer/non-woven cloth and (2) a mixture film of thermal adhesive component and plastic component/non-woven cloth.

6. A heating body according to claim 3, wherein the laminate sheet including the plastic sheet is made of one of:
(1) thermal adhesive plastic component/at least one film selected from mixture films of plastic film, rubber film, plastic component, and rubber component/thermal adhesive plastic component/non-woven cloth;
(2) a mixture film of thermal adhesive plastic component with plastic component/non-woven cloth;
(3) a mixture film of thermal adhesive plastic component with rubber component/non-woven cloth; and
(4) a mixture film of thermal adhesive plastic component, plastic component, and rubber component/non-woven cloth.

7. Aheatingbody according to any one of claims 1 to 3, wherein the heating composition contains an oxidizable metal, an activated carbon, an inorganic electrolyte, water, and a water-retaining polymer agent.

8. A heating body according to claim 3, wherein the heating composition contains an oxidizable metal, an activated carbon, an inorganic electrolyte, water, a thickener, and a water-retaining polymer agent.
